(19) Europäisches Patentamt
European Patent Office
Office européen des brevets

(11) EP 1 900 354 A1

(12) DEMANDE DE BREVET EUROPEEN

(43) Date de publication:
**19.03.2008 Bulletin 2008/12**

(21) Numéro de dépôt: **07111850.9**

(22) Date de dépôt: **05.07.2007**

(51) Int Cl.:
***A61K 8/46*** (2006.01) ***A61Q 5/02*** (2006.01)
***A61Q 19/10*** (2006.01)

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **12.09.2006 FR 0653690**

(71) Demandeur: **L'Oréal**
**75008 Paris (FR)**

(72) Inventeurs:
- **Duranton, Albert**
**78600, Maison Laffite (FR)**
- **Pruche, Francis**
**60300, Senlis (FR)**
- **Lacoutiere, Stéphane**
**75015, Paris (FR)**

(74) Mandataire: **Miszputen, Laurent**
**L'OREAL - D.I.P.I.**
**River Plaza**
**25-29 Quai Aulagnier**
**92665 Asnières-sur-Seine (FR)**

# (54) Procédé d'élimination de l'odeur retenue par les matières kératiniques au contact d'une eau traitée par un désinfectant halogéné

(57) La présente invention a pour objet un procédé cosmétique pour réduire ou éliminer l'odeur retenue par les matières kératiniques mises en contact avec une eau traitée par désinfectant halogéné en particulier un désinfectant chloré et/ou un désinfectant bromé, caractérisé par le fait qu'il consiste à appliquer sur lesdites matières kératiniques au moins une composition contenant dans un milieu cosmétiquement acceptable au moins un composé acide aminoalcalne a sulfonique ou sulfonothioique ou l'un de ses sels, l'un de ses dérivés amides puis à effectuer éventuellement un rinçage à l'eau.

La présente invention a pour objet l'utilisation d'au moins un composé acide aminoalcane sulfonique ou sulfonothioique ou l'un de ses sels, de ses dérivés esters ou amides dans une composition cosmétique , dans le but de réduire ou d'éliminer l'odeur retenue par les matières kératiniques mises en contact avec une eau traitée par un désinfectant halogéné en particulier un désinfectant chloré et/ou un désinfectant bromé.

EP 1 900 354 A1

## Description

La présente invention a pour objet un procédé cosmétique pour réduire ou éliminer l'odeur retenue par les matières kératiniques mise en contact avec une eau traitée par désinfectant halogéné, caractérisé par le fait qu'il consiste à appliquer sur lesdites matières kératiniques au moins une composition contenant dans un milieu cosmétiquement acceptable au moins un composé acide aminoalcalne a sulfonique ou sulfonothioique ou l'un de ses sels, l'un de ses dérivés amides puis à effectuer éventuellement un rinçage à l'eau.

Les piscines sont généralement traitées par des agents désinfectants halogénés notamment des désinfectants chlorés et/ou des désinfectants bromés qui o,t tendance à provoquer une odeur désagréable sur les matières kératiniques telles que la peau et les cheveux perceptible même après plusieurs jours et même après avoir utilisé de manière répétée des lavages au moyen d'un savon, d'un gel douche ou d'un shampooing à la sortie de la piscine.

Le désinfectant le plus généralement utilisé en piscine est le chlore. Quand un désinfectant chloré (acide chlorhydrique et hypochlorite de sodium) est ajouté à l'eau, il prend généralement la forme active d'un acide hypochloreux. L'acide hypochloreux est mesuré dans l'eau en tant que chlore disponible libre.

Les rayonnement UV, un pH bas, les éclaboussures contribuent à la diminution du taux de chlore libre : Le chlore réagit avec l'ammoniaque et l'azote provenant des agents polluants produits par les nageurs tels que la transpiration, l'urine, les huiles solaires pour produire des chloramines combinées.

Les chloramines combinées avec les agents polluants sont malodorantes et sont moins performantes que le chlore libre. Elles peuvent être irritantes pour le corps et les yeux lorqu'elles sont en excès. L'odeur est une indication que le chlore libre a été épuisé et est maintenant sous forme de chloramines combinées.

Il existe le besoin de mettre en oeuvre un procédé permettant de diminuer efficacement voire de supprimer l'odeur retenue par les matières kératiniques lorsque celles-ci sont en contact avec une eau traitée par un composé désinfectant halogéné notamment un désinfectant chloré et/ou un désinfectant bromé.

Certains composés acides aminoalcanes sulfoniques sulfiniques ou sulfonothioiques comme la taurine ou l'homotaurine sont connus pour leurs applications en cosmétique notamment utilisés depuis de nombreuses années dans le domaine du nettoyage de la peau et/ou des cheveux. Ils sont également connus pour le soin capillaire notamment pour la stimulation de la croissance des cheveux dans le brevet FR2668928 ou pour le soin de la peau notamment, pour le traitement des peaux sèches comme dans les demandes JP61145109 et EP1406588.

La demanderesse a maintenant découvert que l'on pouvait sensiblement réduire l'odeur laissée sur les matières kératiniques après mise en contact d'une eau traitée par un désinfectant halogéné en appliquant à la sortie du bain une quantité efficace d'une composition comprenant dans un milieu cosmétiquement acceptable au moins un composé acide aminoalcane sulfonique, sulfinique ou sulfonothioique ou l'un de ses sels, de ses dérivés amides ou ses analogues.

La présente invention a pour objet un procédé cosmétique pour réduire ou éliminer l'odeur retenue par les matières kératiniques mise en contact avec une eau traitée par désinfectant halogéné, caractérisé par le fait qu'il consiste à appliquer sur lesdites matières kératiniques au moins une composition contenant dans un milieu cosmétiquement acceptable au moins un composé acide aminoalcalne sulfonique, sulfonothioique ou sulfiniques ou l'un de ses sels, l'un de ses dérivés amides ou analogues puis à effectuer éventuellement un rinçage à l'eau.

La présente invention a pour objet l'utilisation d'au moins un composé acide aminoalcane sulfonique ou sulfonothioique ou l'un de ses sels, de ses dérivés esters ou amides dans une composition cosmétique , dans le but de réduire ou d'éliminer l'odeur retenue par les matières kératiniques mise en contact avec une eau traitée par un désinfectant halogéné

Toutes ces découvertes sont à la base la présente invention.

Les différents objets de l'invention vont maintenant être détaillés. L'ensemble des significations et définitions des composés utilisés dans la présente invention données ci-dessous sont valables pour l'ensemble des objets de l'invention.

On entend par « désinfectant » tout substance susceptible de détruire les germes microbiens se produisant dans une eau mise en contact avec des matières kératiniques humaines (en particulier peau, cheveux) telle qu'en particulier l'eau d'une piscine, d'un jacuzzi ou les eaux d'adduction.

On entend par « matières kératiniques » au sens de l'invention, la peau, les cheveux, les cils, les sourcils, les lèvres, les ongles, les muqueuses. On entend également les matières kératiniques animales tels que poils et fourrure.

On entend par «milieu cosmétiquement acceptable», un milieu non toxique et susceptible d'être appliqué sur les matières kératiniques. La composition de l'invention peut constituer notamment une composition cosmétique ou dermatologique.

De façon préférentielle, le désinfectant est choisi parmi les désinfectants chlorés et les désinfectants bromés ou leurs mélanges.

Les composés acides aminoalcane sulfoniques, sulfonothioiques ou sulfiniques ainsi que leurs sels ou dérivés amides ou analogues fonctionnels conformes à l'invention sont de préférence choisis parmi les composés répondant à la formule (A) ou (B) suivante :

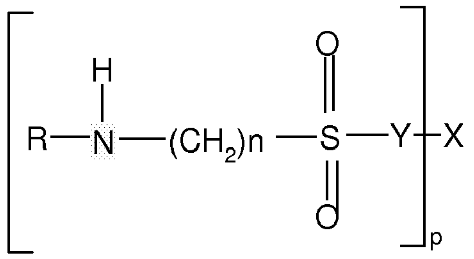

(A)

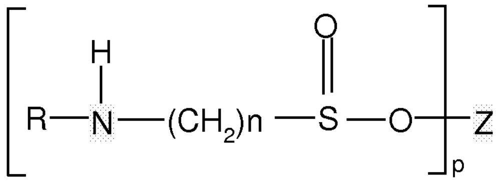

(B)

dans laquelle
R désigne hydrogène, un radical alkyle linéaire ou ramifié en $C_1$-$C_8$ ;
Y désigne S ou O ;
X désigne hydrogène, un cation $M^{p+}$ de valence p, une amine organique
Z désigne hydrogène ou un cation $M^{p+}$ de valence p, une amine organique
n est un entier supérieur ou égal à 2
p est un entier supérieur ou égal à 1

De manière préférentielle,

- R désigne hydrogène ou un radical alkyle linéaire en $C_1$-$C_4$ et plus préférentiellement méthyle.
- p vaut 1 ou 2
- n vaut 2 ou 3 ;
- X désigne hydrogène ou un cation $M^{p+}$ choisi parmi les métaux alcalins (K+, Na+), les alcalino-terreux ($Mg^{2+}$, $Ca^{2+}$), l'ion ammonium.
- Z désigne hydrogène ou un cation $M^{p+}$ choisi parmi les métaux alcalins (K+, Na+), les alcalino-terreux ($Mg^{2+}$, $Ca^{2+}$), l'ion ammonium.

Parmi les composés de formule (A), on peut citer plus particulièrement

- la taurine ou acide 2-aminoethanesulfonique ;
- le taurate de potassium en particulier le taurate de potassium en mélange avec l'acide laurique (nom INCI : Potassium Taurate Laurate) comme le produit commercial L-TK vendu par la société NOF Coropration ;
- le taurate de sodium en particulier en mélange avec l'acide laurique comme le produit commercial L-T2 vendu par la société NOF Corporation ou bien en mélange avec le N-cocoyl N-méthyltaurine comme le produit commercial K-02 vendu par la société NOF Corporation
- le sel de sodium de N-méthyl taurine en particulier sous forme de mélange avec le N-cocoyl N-méthyltaurine (nom INCI : Sodium Methyltaurine Cocoyl Methyl Taurate comme le produit commercial DIAPON K1T2 vendu par la société NOF Corporation;
- la thiotaurine ou acide 2-aminoethanesulfonothioique ;
- l'homotaurine ou acide 2-aminopropanesulfonique ;

Parmi les composés de formule (B), on peut citer plus particulièrement l'hypotaurine ou acide 2-aminoethanesulfinique.

Les composés acides aminoalcane sulfoniques, sulfonothioiques ou sulfiniques ainsi que leurs sels ou dérivés amides conformes à l'invention peuvent également choisis parmi les analogues fonctionnels de la taurine comme ceux décrits dans l'article « Taurine analogues, a new class of therapeutics : retrospect and prospects « Gupta RC, Win T, Bittner S. Curr Med Chem ».

On choisira plus particulièrement la taurine, l'homotaurine ou l'hypotaurine sous forme libre et encore plus particulièrement la taurine.

De manière préférentielle, le ou les composés acides aminoalcane sulfoniques, sulfonothioiques ou sulfiniques ainsi que leurs sels, leurs dérivés amides ou leurs analogues fonctionnels conformes à l'invention sont présents dans la composition à des concentrations allant de 0,05 à 10% et plus préférentiellement de 0,1 à 5% en poids.

Selon une forme particulière de l'invention, les compositions contiennent en outre avantageusement au moins un agent tensioactif qui est généralement présent en une quantité comprise entre 0,1% et 60% en poids environ, de préférence entre 1% et 40% et encore plus préférentiellement entre 5% et 30%, par rapport au poids total de la composition.

Cet agent tensioactif peut être choisi parmi les agents tensioactifs anioniques, amphotères, non-ioniques, ou leurs mélanges.

Les tensioactifs convenant à la mise en oeuvre de la présente invention sont notamment les suivants :

(i) <u>Tensioactif(s) anionique(s)</u> :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$ -oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

(ii) <u>Tensioactif(s) non ionique(s)</u> :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) <u>Tensioactif(s) amphotère(s):</u>

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate

ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits commercialisés sous les dénomination MIRANOL, tels que décrits dans les brevets US-2 528 378 et US-2 781 354 et de structures :

$$R_2\text{-CONHCH}_2\text{CH}_2\text{-N}(R_3)(R_4)(\text{CH}_2\text{COO-}) \qquad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_5\text{-CONHCH}_2\text{CH}_2\text{-N(B)(C)} \qquad (3)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$ - CHOH - $SO_3H$
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHODIA CHIMIE.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélange avec :

- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société COGNIS.

Les compositions de l'invention peuvent également contenir en plus au moins un agent protecteur des matières kératiniques

Les agents protecteurs des matières kératiniques peuvent être tout agent actif utile pour prévenir ou limiter les dégradations dues aux agressions physiques ou chimiques.

Ainsi, l'agent protecteur des matières kératiniques peut être choisi parmi les filtres UV organiques hydrosolubles, liposolubles ou insolubles dans l'eau, les pigments d'oxyde métallique, les agents antiradicalaires, les agents antioxydants, les vitamines, les provitamines.

Les filtres UV (systèmes filtrant les radiations UV) sont notamment choisis parmi les filtres organiques hydrosolubles ou liposolubles, siliconés ou non siliconés et les particules d'oxydes minéraux dont la surface a éventuellement été traitée pour la rendre hydrophile ou hydrophobe.

On peut également utiliser des polymères hydrophiles présentant, en outre et de par leur nature chimique, des propriétés de photoprotection contre le rayonnement UV. On peut citer les polymères comportant des groupements benzylidène camphre et/ou benzotriazole, substitués par des groupements sulfoniques ou ammonium quaternaires.

Les filtres UV organiques hydrophiles, lipophiles ou insolubles sont notamment choisis parmi les anthranilates ; les dérivés de dibenzoylméthane ; les dérivés cinnamiques ; les dérivés salicyliques, les dérivés du camphre ; les dérivés de la benzophénone ; les dérivés de β,β-diphénylacrylate ; les dérivés de triazine ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres UV organiques, on peut citer ceux désignés ci-dessous sous leur nom INCI :

Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane,

Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,

- Diisopropyl Methylcinnamate,

Glyceryl Ethylhexanoate Dimethoxycinnamate

Dérivés de β,β-diphénylacrylate:

Octocrylene vendu notamment sous le nom commercial « UVINUL N539 » par BASF,
Etocrylene, vendu notamment sous le nom commercial « UVINUL N35 » par BASF,

Dérivés de la benzophénone :

Benzophenone-1 vendu sous le nom commercial « UVINUL 400 » par BASF,
Benzophenone-2 vendu sous le nom commercial « UVINUL D50 » par BASF

Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial «MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés de triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
2,4,6-tris(4'-amino benzalmalonate de dinéopentyle)-s-triazine
la 2,4,6-tris-(4'-amino benzalmalonate de diisobutyle)-s- triazine.

- les filtres triazines symétriques décrits dans la demande W02004/085412 (voir composés 6 et 9) notamment les 2,4,6-tris-(biphényl)-1,3,5-triazines (en particulier la 2,4,6-tris(biphenyl-4-yl-1,3,5-triazine) et la 2,4,6-tris(terphenyl)-1,3,5-triazine qui est repris dans les demandes de Beiersdorf WO06/035000, WO06/034982, WO06/034991, WO06/035007, WO2006/034992, WO2006/034985.

Dérivés anthraniliques :

Menthyl anthranilate vendu sous le nom commercial commercial « NEO HELIOPAN MA » par Haarmann et REIMER,

Dérivés d'imidazolines :

Ethylhexyl Dimethoxybenzylidene Dioxoimidazoline Propionate,

Dérivés du benzalmalonate :

Di-néopentyl 4'-méthoxybenzalmalonate
Polyorganosiloxane à fonctions benzalmalonate comme le Polysilicone-15 vendu sous la dénomination commerciale « PARSOL SLX » par HOFFMANN LA ROCHE

Dérivés de 4,4-diarylbutadiène :

- 1,1-dicarboxy (2,2'-diméthyl-propyl)-4,4-diphénylbutadiène

Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V et leurs mélanges.

Les filtres UV organiques préférentiels sont choisis parmi
Ethylhexyl Methoxycinnamate
Homosalate
Ethylhexyl Salicylate,
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s- triazine
la 2,4,6-tris(biphenyl-4-yl-1,3,5-triazine)
la 2,4,6-tris(terphenyl)-1,3,5-triazine
Methylène bis-Benzotriazolyl Tetramethylbutylphénol,
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les oxydes minéraux peuvent être choisis parmi les oxydes de titane, les oxydes de zinc et les oxydes de cérium.

Les agents antioxydants et/ou antiradicalaires sont notamment choisis parmi les phénols tels que le BHA (tert-butyl-4-hydroxyanisole), le BHT (2,6-di-tert-butyl-p-crésol), le TBHQ (tertiobutylhydroquinone), les polyphénols tels que les oligomères proanthocyanidoliques et les flavonoïdes, les amines encombrées connues sous le vocable générique de HALS (Hindered Amine Light Stabilizer) telles que la tétraaminopipéridine, l'acide érythorbique, les polyamines telles que la spermine, la cystéines, le glutathion, la superoxyde dismutase, la lactoferrine.

Les vitamines sont notamment choisies parmi l'acide ascorbique, la vitamine E, l'acétate de vitamine E, les vitamines B telles que les vitamines B3 et B5, la vitamine PP, la vitamine A et ses dérivés.

Les provitamines sont notamment choisies parmi le panthénol et le rétinol.

Selon l'invention, le ou les agents protecteurs des matières kératiniques peuvent représenter de 0,001 % à 20 % en poids, de préférence de 0,01 % à 10% en poids et plus particulièrement de 0,1 à 5% en poids par rapport au poids total de la composition finale.

Les compositions de l'invention peuvent également contenir en plus au moins un agent conditionneur.

Dans le cadre de la présente demande, on entend par « agent conditionneur », tout agent ayant pour fonction l'amélioration des propriétés cosmétiques de la peau ou des cheveux, en particulier la douceur, le démêlage, le toucher, le lissage, l'électricité statique.

Les agents de conditionnement peuvent se présenter sous forme liquide, semi-solide ou solide tels que par exemple des huiles, des cires ou des gommes.

Selon l'invention, les agents conditionneurs peuvent être choisis parmi les huiles de synthèses telles que les poly-oléfines, les huiles végétales, les huiles fluorées ou perfluorées, les cires naturelles ou synthétiques, les silicones,

les polymères cationiques, les protéines ou hydrolysats de protéines cationiques , les composés de type céramide, les tensioactifs cationiques, les amines grasses, les acides gras et leurs dérivés ainsi que les mélanges de ces différents composés.

Les agents conditionneurs préférés selon l'invention sont les polymères cationiques et les silicones.

Les huiles de synthèse sont notamment les polyoléfines en particulier les poly-$\alpha$-oléfines et plus particulièrement :

- de type polybutène, hydrogéné ou non, et de préférence polyisobutène, hydrogéné ou non.

On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.

A titre d'exemples de poly-$\alpha$-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),

- de type polydécène, hydrogéné ou non.

De tels produits sont vendus par exemple sous les dénominations ETHYLFLO par la société ETHYL CORP., et d'ARLAMOL PAO par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule $R_9COOR_{10}$ dans laquelle $R_9$ représente le reste d'un acide gras supérieur comportant de 7 à 29 atomes de carbone et $R_{10}$ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;

On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français 2 505 348 ou 2 542 997. Parmi ces polymères, on peut citer :

- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthyl-ammonium décrit par exemple dans la demande de brevet EP-A-080976 et vendus sous la dénomination BINA QUAT P 100 par la société CIBA GEIGY,

- le copolymère d'acrylamide et de méthosulfate de méthacryloyloxyéthyltriméthyl-ammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français FR 2 077 143 et 2 393 573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymères vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP.

Conviennent également à titre de polymères cationiques, les polysaccharides cationiques notamment les celluloses à l'image par exemple des polymères commercialisés sous les dénominations "JR" (JR 400, JR 125, JR 30M) ou "LR" (LR 400, LR 30M) par la Société AMERCHOL et les produits commercialisés sous la dénomination "Celquat L 200" et "Celquat H 100" par la Société National Starch et les gommes de galactomannanes cationiques comme par exemple les produits commercialisés notamment sous les dénominations commerciales de JAGUAR C13 S, JAGUAR C 15, JAGUAR C 17 ou JAGUAR C162 par la société RHODIA.

Les polymères cationiques convenant à l'invention peuvent également être choisis parmi :

- les dérivés de polyaminoamides comme par exemple les polymères acide adipique/diméthylaminohydroxypropyl/diéthylène triamine vendus sous la dénomination "Cartaretine F, F4 ou F8" par la société Sandoz,
- les polymères dérivant de polyalkylène polyamine comme par exemple ceux commercialisés sous la dénomination "Hercosett 57" par la société Hercules Inc. ou bien sous la dénomination de "PD 170" ou "Delsette 101 " par la société Hercules dans le cas du copolymère d'acide adipique/époxypropyl/diéthylène-triamine.
- les cyclopolymères d'alkyl diallyl amine ou de dialkyl diallyl ammonium tels que les polymères vendus sous la dénomination "MERQUAT 100" par la société NALCO (et leurs homologues de faibles masses molaires moyenne en poids) et les copolymères de sels (par exemple chlorure) de diallyldiméthylammonium et d'acrylamide par exemple commercialisés sous la dénomination "MERQUAT 550",
- les polymères de diammonium ou de polyammonium quaternaires comme par exemple les produits "Mirapol® A 15", "Mirapol® AD1 ", "Mirapol® AZ1" et "Mirapol® 175" vendus par la société Miranol,
- les polymères quaternaires de vinylpyrrolidone et de vinylimidazole tels que par exemple les produits commercialisés sous les dénominations Luviquat® FC 905, FC 550 et FC 370 par la société B.A.S.F,
- les polyamines comme le Polyquart® H vendu par COGNIS, référencé sous le nom de "POLYETHYLENEGLYCOL (15) TALLOW POLYAMINE" dans le dictionnaire CTFA, et
- les polymères de préférence réticulés de sels de méthacryloyloxyalkyl(C1-C4) trialkyl(C1-C4)ammonium tels que par exemple les produits commercialisés à l'état de dispersion sous le nom de "SALCARE® SC 92", de "SALCARE® SC 95" et "SALCARE® SC 96" par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination "JR 400" par la Société AMERCHOL, les cyclopolymères cationiques, en particulier les homopolymères ou copolymères de sels (par exemple chlorure) de diméthyldiallylammonium connus sous le nom INCI « Polyquaternium-7 » comme les produits vendus sous les dénominations "MERQUAT 100", "MERQUAT 550" et "MERQUAT S" par la société NALCO et leurs homologues de faibles poids moléculaires en poids, les polysaccharides cationiques tels que les gommes de guar modifiées par un sel de 2,3-époxypropyl triméthylammonium, les polymères quaternaires de vinylpyrrolidone et de vinylimidazole, les homopolymères ou copolymères éventuellement réticulés de sels de méthacryloyloxyalkyl(C1-C4) trialkyl(C1-C4)ammonium et leurs mélanges.

Le ou les polymères cationiques sont généralement présents à des concentrations allant de 0,01 à 20 %, de préférence de 0,05 à 10 % et plus particulièrement de 0,1 à 5 % en poids par rapport au poids total de la composition.

A titre de silicones utilisables dans les compositions de la présente invention, on peut notamment citer les silicones volatiles ou non, cycliques ou acycliques, ramifiées ou non, organomodifiées ou non, telles que décrites ci-dessous.

Les silicones utilisables conformément à l'invention peuvent être solubles ou insolubles dans la composition et en particulier être des polyorganosiloxanes insolubles dans la composition de l'invention ; elles peuvent se présenter sous forme d'huiles, de cires, de résines ou de gommes.

Selon l'invention, toutes les silicones peuvent être utilisées telle quelle ou sous forme de solutions, de dispersions, d'émulsions, de nanoémulsions ou de microémulsions.

Les organopolysiloxanes sont définis plus en détail dans l'ouvrage de Walter NOLL "Chemistry and Technology of Silicones" (1968) Academie Press. Elles peuvent être volatiles ou non volatiles.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10-6 à 2,5 m2/s à 25 °C et de préférence 1.10-5 à 1 m2/s. La viscosité des silicones est, par exemple, mesurée à 25 °C selon la norme ASTM 445 Appendice C.

Parmi ces polyalkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple, l'huile 70 047 V 500 000 ;
- les huiles de la série MIRASIL® commercialisées par la société RHODIA ;
- les huiles de la série 200 de la société DOW CORNING telles que plus particulièrement la DC200 de viscosité 60 000 mm2/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol, connus sous le nom de dimethiconol (CTFA), tels que les huiles de la série 48 de la société RHODIA .

Dans cette classe de polyalkylsiloxanes, on peut également citer les produits commercialisés sous les dénominations "ABIL WAX® 9800 et 9801" par la société GOLDSCHMIDT qui sont des polyalkyl(C1-C20)-siloxanes.
Il peut s'agir de polyalkylarylsiloxanes, notamment choisis parmi les polydiméthyl/méthylphénylsiloxanes, les polydiméthyldiphényl-siloxanes linéaires et/ou ramifiés de viscosité de 1.10-5 à 5.10-2 m2/s à 25 °C.

Parmi ces polyalkylarylsiloxanes on peut citer, à titre d'exemple, les produits commercialisés sous les dénominations suivantes :

- les huiles SILBIONE® de la série 70 641 de RHODIA ;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Des produits plus particulièrement utilisables conformément à l'invention sont des mélanges tels que :

- les mélanges formés à partir d'un polydiméthylsiloxane hydroxylé en bout de chaîne ou diméthiconol (CTFA) et d'un poly-diméthylsiloxane cyclique également appelé cyclométhicone (CTFA) tel que le produit Q2 1401 commercialisé par la société DOW CORNING ;
- les mélanges formés à partir d'une gomme polydiméthyl-siloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m2/s et d'une huile SF 96 d'une viscosité de 5.10-6 m2/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

$R_2SiO_2/_2$, $R_3SiO_1/_2$, $RSiO_3/_2$ et $SiO_4/_2$ dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C1-C4, plus particulièrement méthyle, ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthylsiloxane.

On peut également citer les résines du type triméthylsiloxysilicate commercialisées notamment sous les dénominations X22-4914, X21-5034 et X21-5037 par la société SHIN-ETSU.

Les silicones telles que décrites ci-dessus peuvent être utilisées seules ou en mélange, en une quantité comprise entre 0,01 et 20 % en poids, de préférence entre 0,1 et 5 % en poids par rapport au poids total de la composition.

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :

- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société MAYBROOOK et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres:

- le "Croquat L" dont les groupements ammonium quaternaires comportent un groupement alkyle en $C_{12}$;
- le "Croquat M" dont les groupements ammonium quaternairees comportent des groupements alkyle en $C_{10}$-$C_{18}$ ;
- le "Croquat S" dont les groupements ammonium quaternaires comportent un groupement alkyle en $C_{18}$ ;
- le "Crotein Q" dont les groupements ammonium quaternaires comportent au moins un groupe alkyle ayant de 1 à 18 atomes de carbone.
  Ces différents produits sont vendus par la Société Croda.

D'autre protéines ou hydrolysats quaternisés sont par exemple ceux répondant à la formule (XIV) :

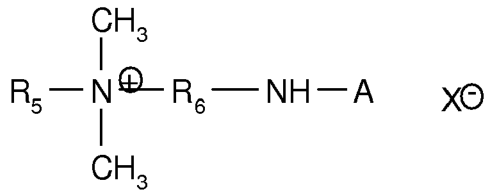
(XIV)

dans laquelle $X^-$ est un anion d'un acide organique ou minéral, A désigne un reste de protéine dérivé d'hydrolysats de protéine de collagène, $R_5$ désigne un groupement lipophile comportant jusqu'à 30 atomes de carbone, $R_6$ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Selon la présente invention, les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :

- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique.
- le N-docosanoyl N-méthyl-D-glucamine

ou les mélanges de ces composés.

On peut également utiliser des tensioactifs cationiques parmi lesquels on peut citer en particulier : les sels d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Les sels d'ammonium quaternaires sont par exemple :

- ceux qui présentent la formule générale (XV) suivante:

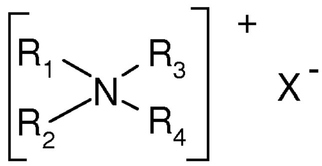

(XV)

dans laquelle les radicaux $R_1$ à $R_4$, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène ($C_2$-$C_6$), alkylamide, alkyl($C_{12}$-$C_{22}$)amido alkyle($C_2$-$C_6$), alkyl($C_{12}$-$C_{22}$)acétate, hydroxyalkyle, comportant environ de 1 à 30 atomes de carbone; X est un anion choisi dans le groupe des halogénures, phosphates, acétates, lactates, alkyl($C_2$-$C_6$)sulfates, alkyl-ou-alkylarylsulfonates,

- les sels d'ammonium quaternaire de l'imidazolinium, comme par exemple celui de formule (XVI) suivante :

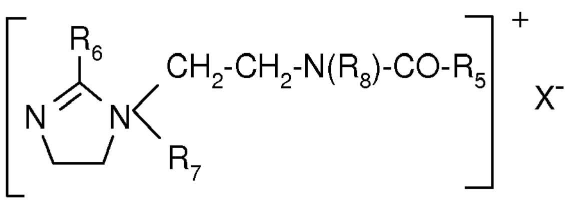

(XVI)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en $C_1$-$C_4$ , $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple commercialisé sous la dénomination «REWOQUAT W 75» par la société DEGUSSA,

- les sels de diammonium quaternaire de formule (XVII) :

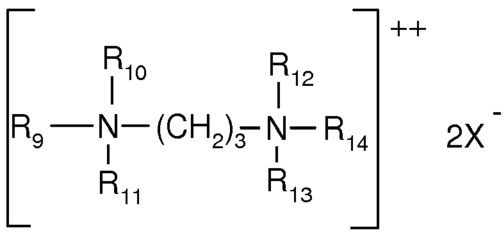

(XVII)

dans laquelle $R_9$ désigne un radical aliphatique comportant environ de 16 à 30 atomes de carbone, $R_{10}$, $R_{11}$, $R_{12}$, $R_{13}$ et $R_{14}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

- les sels d'ammonium quaternaire contenant au moins une fonction ester

Les sels d'ammonium quaternaire contenant au moins une fonction ester utilisables selon l'invention sont par exemple ceux de formule (XVIII) suivante :

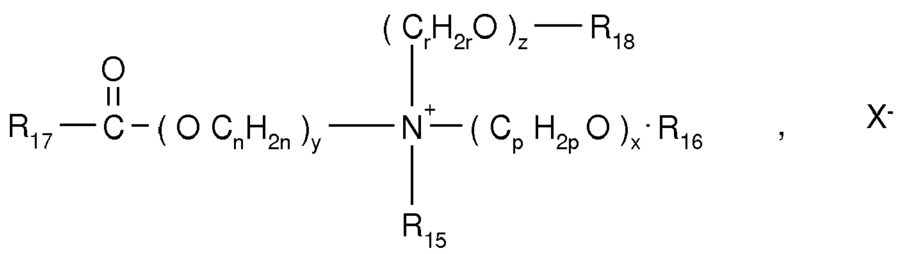

(XVIII)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :
  - le radical

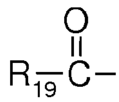

  - les radicaux $R_{20}$ hydrocarbonés en $C_1$-$C_{22}$ linéaires ou ramifiés, saturés ou insaturés,
  - l'atome d'hydrogène,
- $R_{18}$ est choisi parmi :
  - le radical

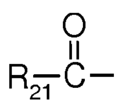

  - les radicaux $R_{22}$ hydrocarbonés en $C_1$-$C_6$ linéaires ou ramifiés, saturés ou insaturés,
  - l'atome d'hydrogène,

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés;
- n, p et r, identiques ou différents, sont des entiers valant de 2 à 6 ;
- y est un entier valant de 1 à 10 ;
- x et z, identiques ou différents, sont des entiers valant de 0 à 10 ;
- $X^-$ est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

Les radicaux alkyles $R_{15}$ peuvent être linéaires ou ramifiés et plus particulièrement linéaires.

De préférence $R_{15}$ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque $R_{16}$ est un radical $R_{20}$ hydrocarboné, il peut être long et avoir de 12 à 22 atomes de carbone ou court et avoir de 1 à 3 atomes de carbone.

Lorsque $R_{18}$ est un radical $R_{22}$ hydrocarboné, il a de préférence 1 à 3 atomes de carbone.

Avantageusement, $R_{17}$, $R_{19}$ et $R_{21}$, identiques ou différents, sont choisis parmi les radicaux hydrocarbonés en $C_{11}$-$C_{21}$, linéaires ou ramifiés, saturés ou insaturés, et plus particulièrement parmi les radicaux alkyle et alcényle en $C_{11}$-$C_{21}$, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion $X^-$ est encore plus particulièrement le chlorure ou le méthylsulfate.

On utilise plus particulièrement les sels d'ammonium de formule (XVIII) dans laquelle :

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi :
    - le radical

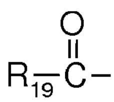

    - les radicaux méthyle, éthyle ou hydrocarbonés en $C_{14}$-$C_{22}$
    - l'atome d'hydrogène ;
- $R_{18}$ est choisi parmi :
    - le radical

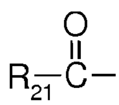

    - l'atome d'hydrogène ;

$R_{17}$, $R_{19}$ et $R_{21}$, identiques ou différents, sont choisis parmi les radicaux hydrocarbonés en $C_{13}$-$C_{17}$, linéaires ou ramifiés, saturés ou insaturés et de préférence parmi les radicaux alkyles et alcényle en $C_{13}$-$C_{17}$, linéaires ou ramifiés, saturés ou insaturés.

Avantageusement, les radicaux hydrocarbonés sont linéaires.

On peut citer par exemple les composés de formule (XVI) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme

ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUAT WE 18 par la société DEGUSSA.

On peut aussi utiliser les sels d'ammonium contenant au moins une fonction ester décrits dans les brevets US-A-4874554 et US-A-4137180.

Parmi les sels d'ammonium quaternaire de formule (XV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Les acides gras sont choisis plus particulièrement parmi l'acide myristique, l'acide palmitique, l'acide stéarique, l'acide béhénique, l'acide oléïque, l'acide linoléïque, l'acide linolénique et l'acide isostéarique,

Les dérivés d'acides gras sont notamment les esters d'acides carboxyliques en particulier les esters mono, di, tri ou tétracarboxyliques.

Les esters d'acides monocarboxyliques sont notamment les monoesters d'acides aliphatiques saturés ou insaturés, linéaires ou ramifiés en $C_1$-$C_{26}$ et d'alcools aliphatiques saturés ou insaturés, linéaires ou ramifiés en $C_1$-$C_{26}$ , le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle, le néopentanoate d'isostéaryle, le néopentanoate d'isodécyle.

On peut également utiliser les esters d'acides di ou tricarboxyliques en C4-C22 et d'alcools en $C_1$-$C_{22}$ et les esters d'acides mono di ou tricarboxyliques et d'alcools di, tri, tétra ou pentahydroxy en $C_2$-$C_{26}$.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle, le néopentanoate d'isostéaryle, le néopentanoate d'isodécyle.

Les huiles fluorées sont par exemple les perfluoropolyéthers décrits notamment dans la demande de brevet EP-A-486135 et les composés fluorohydrocarbonées décrites notamment dans la demande de brevet WO 93/11103.

L'enseignement de ces deux demandes est totalement inclus dans la présente demande à titre de référence.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Il est bien entendu possible de mettre en oeuvre des mélanges d'agents conditionneur.

Selon l'invention, le ou les agents conditionneurs peuvent représenter de 0,001 % à 20 % en poids, de préférence de 0,01 % à 10% en poids et plus particulièrement de 0,1 à 3% en poids par rapport au poids total de la composition finale.

Le milieu aqueux cosmétiquement acceptable des compositions de l'invention peut contenir, outre l'eau, un ou plusieurs solvants choisis parmi les alcools inférieurs comportant de 1 à 6 atomes de carbone, tels que l'éthanol; les polyols tels que la glycérine ; les glycols comme le butylène glycol, l'isoprène glycol, le propylène glycol, le dipropylèneglycol, l'hexylèneglycol (2-méthyl 2,4-pentanediol), le néopentylglycol et le 3-méthyl-1,5-pentanediol; les polyéthylène glycols tels que le PEG-8; le sorbitol ; les sucres tels que le glucose, le fructose, le maltose, le lactose, le sucrose ; les éthers de polyols tels que, par exemple, les éthers monométhylique, monoéthylique et monobutylique d'éthylèneglycol, le monométhyléther de propylèneglycol,; ainsi que les alkyléthers de diéthylèneglycol, notamment en C1-C4, comme par exemple, le monoéthyléther ou le monobutyléther du diéthylèneglycol et leurs mélanges.

La quantité de solvant(s) dans la composition de l'invention peut aller de 0,5 à 40 % en poids et de préférence de 5 à 20 % en poids par rapport au poids total de la composition. De préférence, la composition comprend de 50 à 95 % en poids d'eau par rapport au poids total de la composition.

Les compositions de l'invention peuvent comprendre des adjuvants habituellement utilisés dans le domaine cosmétique, et notamment ceux utilisés dans les produits de nettoyage. Comme adjuvants, on peut citer par exemple les parfums, les conservateurs, les séquestrants (EDTA), les pigments, les agents nacrants ou opacifiants, les charges minérales ou organiques, matifiantes, blanchissantes ou exfoliantes, les colorants solubles, les actifs cosmétiques ou dermatologiques, les polymères non ioniques tels que le polyvinylpyrrolidone (PVP), les polymères anioniques, les corps gras incompatibles avec le milieu aqueux, comme les huiles ou les cires. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme exemple d'huile, on peut citer les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles.

Comme actifs, on peut utiliser dans la composition de l'invention tout actif habituellement utilisé dans les domaines cosmétique et dermatologique, tels que par exemple les vitamines ou provitamines hydrosolubles ou liposolubles comme les vitamines A (rétinol), C (acide ascorbique), B3 ou PP (niacinamide), B5 (panthénol), E (tocophérol), K1, le bêta-carotène, et les dérivés de ces vitamines et notamment leurs esters ; les stéroïdes comme la DHEA et la 7$\alpha$-hydroxy DHEA ; les antiseptiques ; les antisébohrréïques et antimicrobiens tels que le peroxyde de benzoyle, l'acide salicylique, le triclosan, le tricarban, l'acide azélaïque ; les hydratants comme la glycérine, l'acide hyaluronique, le pyrrolidone carboxylique acide (PCA) et ses sels, le pidolate de sodium, la sérine, le xylitol, le tréhalose, l'ectoïne, les céramides, l'urée ; les agents kératolytiques et anti-âge tels que les alpha-hydroxyacides comme l'acide glycolique, l'acide citrique, l'acide lactique, les béta-hydroxyacides comme l'acide salicylique et ses dérivés; les enzymes et coenzymes et notamment le coenzyme Q10 ; les filtres solaires; les azurants optiques ; les actifs amincissants comme la caféine, la théophyline, la théobromine, les anti-inflammatoires tels que les acides 18 $\beta$ glycyrrhétinique et ursolique, et leurs mélanges. On peut utiliser un mélange de deux ou plusieurs de ces actifs. Le ou les actifs peuvent être par exemple présents en une concentration allant de 0,01 à 20 %, de préférence de 0,1 à 10 % et mieux de 0,5 à 5 % du poids total de la composition.

Comme charges, on peut citer les charges minérales telles que le talc ou silicate de magnésium (granulométrie: 5 microns) commercialisé sous la dénomination LUZENAC 15 M00® par la société Luzenac, le kaolin ou silicate d'aluminium comme par exemple celui commercialisé sous la dénomination KAOLIN SUPREME® par la société Imerys, ou les charges organiques telles que l'amidon comme par exemple le produit commercialisé sous la dénomination AMIDON DE MAIS B ® par la société Roquette, les microsphères de Nylon comme celles commercialisées sous la dénomination ORGASOL 2002 UD NAT COS® par la société Atochem, les microsphères à base de copolymère de chlorure de

vinylidene/Acrylonitrile/methacrylonitrile enfermant de l'isobutane, expansées comme celles commercialisées sous la dénomination EXPANCEL 551 DE® par la société Expancel. On peut ajouter aussi à la composition de l'invention, des fibres comme par exemple des fibres de nylon (POLYAMIDE 0.9 DTEX 0.3 MM commercialisé par les Etablissements Paul Bonte), des fibres de cellulose ou « Rayonne » (RAYON FLOCK RCISE NOOO3 MO4® commercialisé par la société Claremont Flock Corporation).

Les compositions conformes à l'invention peuvent être plus particulièrement utilisées pour le lavage ou le traitement des matières kératiniques en particulier la peau et les cheveux.

En particulier, les compositions selon l'invention sont des compositions détergentes telles que des shampooings, des gels-douches et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale. De préférence, la base lavante contient au moins 3% en poids de tensioactif anioniques, plus particulièrement de 4 à 30% en poids par rapport au poids total de la composition.

Le pH de la composition appliquée sur les matières kératiniques est généralement compris entre les valeurs 2 et 11. Il est de préférence compris entre 3 et 8, et peut être ajusté à la valeur désirée au moyen d'agents acidifiants ou alcalinisants bien connus de l'état de la technique des compositions appliquées sur des matières kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XX) suivante :

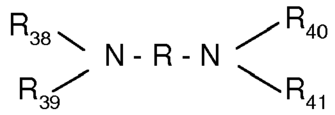 (XX)

dans laquelle R est un reste propylène éventuellement substitué par un groupement hydroxyle ou un radical alkyle en $C_1$-$C_4$ $R_{38}$, $R_{39}$, $R_{40}$ et $R_{41}$, identiques ou différents, représentent un atome d'hydrogène, un radical alkyle en $C_1$-$C_4$ ou hydroxyalkyle en $C_1$-$C_4$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Les compositions de l'invention peuvent également se présenter sous forme d'après-shampooing à rincer ou non.

Les compositions de l'invention peuvent encore se présenter sous la forme de compositions lavantes pour la peau, et en particulier sous la forme de solutions ou de gels pour le bain ou la douche ; sous forme de savons solides.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray ou une mousse pour le traitement de la peau ou des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.

**<u>Exemple 1 :</u> Test de neutralisation d'odeur d'une eau traitée avec un agent désinfectant chloré:**

**[0142]** - Eau + agent chlorant pour piscine du commerce (ref Bayrol) à 10 g/$m^3$, à cette concentration X 10, à cette concentration X 100.

Dans chacune de ces eaux contenue dans un récipient, on ajoute à volume égal soit du tampon phosphate sans taurine soit du tampon phosphate associé à 1% en poids de taurine dans ledit tampon.
L"odeur est évaluée par 6 personnes en aveugle. On demande à chacun la comparaison des différents échantillons contenant l'agent chlorant à la même concentration et avec ou sans taurine.
De manière unanime, les échantillons contenant la taurine dégagent une odeur moindre que ceux sans taurine.

**Exemple 2 : Test comparatif entre deux gels douches 1 et 2**

On réalise les deux gels douches suivants (les quantité sont exprimées en pourcentages en poids) :

| Ingrédients (NOM INCI) | Gel douche 1 (invention) | Gel douche 2 (hors invention |
|---|---|---|
| SODIUM CHLORIDE | 2,4 | 2,5 |
| TAURINE | 1 | 0 |
| CITRIC ACID | 0.2 | 0.2 |
| DMDM HYDANTOIN | 0,2 | 0,2 |
| SODIUM METHYLPARABEN | 0,25 | 0,25 |
| POLYQUATERNIUM-7 | 0,1 | 0,1 |
| AQUA | 72,9 | 73,8 |
| GLYCERIN | 2 | 2 |
| COCO-BETAINE | 1,5 | 1,5 |
| SODIUM LAURETH SULFATE | 10,5 | 10,5 |

On évalue leur activité déodorante sur les jambes d'un panel de 15 personnes de type européen présentant une pilosité abondante ou normale au niveau des jambes (mollet) selon le protocole suivant :

- le bain est réalisé dans le jacuzzi avec une température de l'eau à 40 °C et avec remous. La quantité de chlore mise dans le bain est de 3mg/l. Le pH de l'eau après solubilisation du chlore est de 7.8.
- le temps d'immersion des jambes : 15 minutes
- chaque jambe est humidifiée ( température 38 °C et débit de l'eau dans les conditions d'usage) , le gel douche 1 est appliqué à la quantité de 1.2 g sur la jambe droite ( du pied au genou) puis massé pendant 1 minute. Pause du produit pendant 1 minute et rinçage.
- essuyage de chaque jambe avec une serviette jetable ( 1 serviette/jambe)
- temps de pause: 15 minutes
- séchage au sèche-cheveux pendant 20 secondes avant le sniff
- sniff direct réalisé par 2 experts sur l'échelle suivante :

**ECHELLE D'INTENSITE DU CHLORE GLOBALE : (I. G.)**

| 0 | 1 | 2 | 3 | 4 |
|---|---|---|---|---|
| Nulle | Légère | Modérée | Forte | Très Forte |

On mesure la valeur moyenne d'intensité de l'odeur du chlore ainsi que le pourcentage de variation de l'intensité de l'odeur par le calcul suivant

% variation = (intensité du gel 1 – intensité du gel 2 )X100 / intensité du gel 2

On effectue un test statistique selon le test de Wilcoxon.

**RESULTATS**

| Composition | Gel douche 1 (invention) | Gel douche 2 (hors invention |
|---|---|---|
| **Intensité de l'odeur** | 1,26 ± 0.2 | 1,75± 0.17 |
| **% de variation** | **28%** | - |
| **Significativité du test** | **oui** | |

Les résultats montrent que l'intensité de l'odeur de chlore est plus faible sur une jambe traitée par un gel douche 1 selon l'invention contenant la taurine comparativement à une jambe lavée avec le gel douche 2 sans taurine.

**Revendications**

1. Procédé cosmétique pour réduire ou éliminer l'odeur retenue par les matières kératiniques mises en contact avec une eau traitée par désinfectant halogéné, **caractérisé par le fait qu'**il consiste à appliquer sur lesdites matières kératiniques au moins une composition contenant, dans un milieu cosmétiquement acceptable, au moins un composé acide aminoalcalne, sulfonique ou sulfonothioique ou l'un de ses sels, l'un de ses dérivés amides ou l'un de ses analogues fonctionnels puis à effectuer éventuellement un rinçage à l'eau.

2. Procédé selon la revendication 1, où le désinfectant halogéné est choisi parmi les désinfectants chlorés et les désinfectants bromés ou leurs mélanges.

3. Procédé selon la revendication 1 ou 2, où le composé acide aminoalcane sulfonique, sulfonothioique ou sulfinique ou ses sels ou dérivés amides sont choisis parmi les composés répondant à la formule (A) ou (B) suivante :

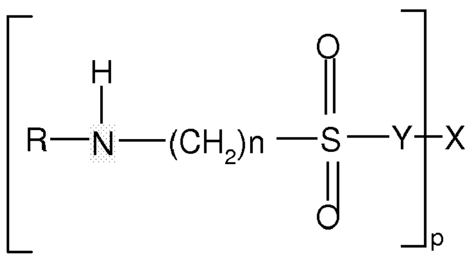

(A)

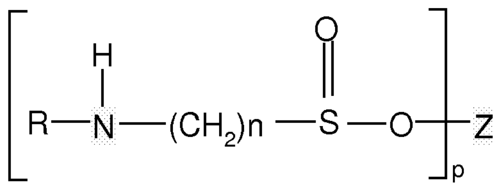

(B)

dans laquelle
R désigne hydrogène, un radical alkyle linéaire ou ramifié en $C_1$-$C_8$ ;
Y désigne S ou O ;
X désigne hydrogène, un cation $M^{p+}$ de valence p, une amine organique
Z désigne hydrogène ou un cation $M^{p+}$ de valence p, une amine organique
n est un entier supérieur ou égal à 2

p est un entier supérieur ou égal à 1

**4.** Procédé selon la revendication 3, où les conditions suivantes sont remplies

- R désigne hydrogène ou un radical alkyle linéaire en $C_1$-$C_4$ et plus préférentiellement méthyle.
- p vaut 1 ou 2
- n vaut 2 ou 3 ;
- X désigne hydrogène ou un cation $M^{p+}$ choisi parmi les métaux alcalins (K+, Na+), les alcalino-terreux (Mg $^{2+}$, Ca $^{2+}$), l'ion ammonium.
- Z désigne hydrogène ou un cation $M^{p+}$ choisi parmi les métaux alcalins (K+, Na+), les alcalino-terreux (Mg $^{2+}$, Ca $^{2+}$), l'ion ammonium.

**5.** Procédé selon l'une quelconque des revendications 1 à 4, où le composé acide aminoalcane sulfonique, sulfonothioique ou sulfinique ou ses sels ou dérivés amides sont choisis parmi les composés suivants :

- la taurine ou acide 2-aminoethanesulfonique ;
- le taurate de potassium
- le taurate de sodium
- le sel de sodium de N-méthyl taurine
- la thiotaurine ou acide 2-aminoethanesulfonothioique ;
- l'homotaurine ou acide 2-aminopropanesulfonique ;
- l'hypotaurine ou acide 2-aminoethanesulfinique.

**6.** Procédé selon l'une quelconque des revendications 1 à 5, où l'on utilise la taurine, l'homotaurine ou l'hypotaurine sous forme libre.

**7.** Procédé selon l'une quelconque des revendications 1 à 6, où le ou les composés acides aminoalcane sulfoniques, sulfonothioiques ou sulfiniques ainsi que leurs sels ou dérivés amides conformes à l'invention sont présents dans la composition à des concentrations allant de 0,05 à 10% et plus préférentiellement de 0,1 à 5% en poids par rapport au poids total de la composition.

**8.** Procédé selon l'une quelconque des revendications 1 à 7, où la composition contient en outre au moins un agent tensioactif.

**9.** Procédé selon l'une quelconque des revendications 1 à 8, où la composition contient en plus au moins un agent protecteur des matières kératiniques

**10.** Procédé selon l'une quelconque des revendications 1 à 9, où la composition contient en plus au moins au moins un agent conditionneur.

**11.** Procédé selon la revendication 10, où le conditionneur est choisi parmi les polymères cationiques et les silicones.

**12.** Procédé selon la revendication 10, où le conditionneur est choisi parmi les dérivés d'éther de cellulose quaternaires ; les homopolymères ou copolymères de sels de diméthyldiallylammonium ; les gommes de guar modifiées par un sel de 2,3-époxypropyl triméthylammonium ; les polymères quaternaires de vinylpyrrolidone et de vinylimidazole, les homopolymères ou copolymères éventuellement réticulés de sels de méthacryloyloxyalkyl(C1-C4) trialkyl(C1-C4)ammonium et leurs mélanges.

**13.** Procédé selon l'une quelconque des revendications 1 à 12, où la composition contient en plus au moins un additif choisi parmi les parfums, les conservateurs, les séquestrants (EDTA), les pigments, les agents nacrants ou opacifiants, les charges minérales ou organiques, matifiantes, blanchissantes ou exfoliantes, les colorants solubles, les actifs cosmétiques ou dermatologiques, les polymères non ioniques tels que le polyvinylpyrrolidone (PVP), les polymères anioniques, les corps gras incompatibles avec le milieu aqueux, comme les huiles ou les cires.

**14.** Procédé selon l'une quelconque des revendications 1 à 13, où la composition contient une base lavante représentant de 4 % à 50 % en poids, de préférence de 6 % à 35 % en poids, et encore plus préférentiellement de 8 % à 25 % en poids, du poids total de la composition finale.

**15.** Procédé selon la revendication 14, où la base lavante contient au moins 3% en poids de tensioactif anionique, plus particulièrement de 4 à 30% en poids par rapport au poids total de la composition.

**16.** Procédé selon l'une quelconque des revendications 1 à 15, où le pH de la composition appliquée sur les matières kératiniques est généralement compris entre les valeurs 2 et 11 et de préférence compris entre 3 et 8.

**17.** Utilisation d'au moins un composé acide aminoalcane sulfonique ou sulfonothioique ou l'un de ses sels, ou l'un de ses dérivés amides ou ou l'un de ses analogues fonctionnels tel que défini dans l'une quelconque des revendications précédentes dans une composition cosmétique, dans le but de réduire ou d'éliminer l'odeur retenue par les matières kératiniques mise en contact avec une eau traitée par un désinfectant halogéné en particulier un désinfectant chloré et/ou un désinfectant bromé.

Office européen des brevets

# RAPPORT DE RECHERCHE EUROPEENNE

Numéro de la demande
EP 07 11 1850

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2003/012761 A1 (YOSHIDA KATSUNORI [JP] ET AL) 16 janvier 2003 (2003-01-16)<br>* exemples * | 1-17 | INV.<br>A61K8/46<br>A61Q5/02<br>A61Q19/10 |
| A | FR 2 841 101 A1 (OREAL [FR]) 26 décembre 2003 (2003-12-26)<br>* page 3, ligne 5 - ligne 9 *<br>* exemples * | 1-17 | |
| A | US 2001/034318 A1 (YAMAMOTO HIROSHI [JP] ET AL) 25 octobre 2001 (2001-10-25)<br>* le document en entier * | 1-17 | |
| D,A | JP 61 145109 A (NAKANISHI EIKO; AKIYOSHI MASATAKA) 2 juillet 1986 (1986-07-02)<br>* abrégé * | 1-17 | |
| D,A | WO 03/005979 A (BEIERSDORF AG [DE]; SAUERMANN GERHARD [DE]) 23 janvier 2003 (2003-01-23)<br>* page 1, alinéa 1 *<br>* page 9, ligne 4 - ligne 18 *<br>* exemples 1-3 * | 1-17 | DOMAINES TECHNIQUES RECHERCHES (IPC)<br>A61K<br>A61Q |

Le présent rapport a été établi pour toutes les revendications

2

| Lieu de la recherche | Date d'achèvement de la recherche | Examinateur |
|---|---|---|
| Munich | 5 décembre 2007 | Ruckebusch, Virginie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 07 11 1850

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

05-12-2007

| Document brevet cité au rapport de recherche | Date de publication | Membre(s) de la famille de brevet(s) | Date de publication |
|---|---|---|---|
| US 2003012761 A1 | 16-01-2003 | CN 1383377 A | 04-12-2002 |
| | | EP 1295591 A1 | 26-03-2003 |
| | | WO 0200179 A1 | 03-01-2002 |
| FR 2841101 A1 | 26-12-2003 | AUCUN | |
| US 2001034318 A1 | 25-10-2001 | AUCUN | |
| JP 61145109 A | 02-07-1986 | AUCUN | |
| WO 03005979 A | 23-01-2003 | DE 10133202 A1 | 16-01-2003 |
| | | EP 1406588 A2 | 14-04-2004 |
| | | JP 2004536838 T | 09-12-2004 |
| | | US 2004220137 A1 | 04-11-2004 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2668928
- JP 61145109 B
- EP 1406588 A
- US 2528378 A
- US 2781354 A
- US 5624663 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 2004085412 A
- WO 06035000 A
- WO 06034982 A
- WO 06034991 A
- WO 06035007 A
- WO 2006034992 A
- WO 2006034985 A
- EP 0337354 A
- FR 2270846 A
- FR 2383660 A
- FR 2598611 A
- FR 2470596 A
- FR 2519863 A
- FR 2505348
- FR 2542997
- EP 080976 A
- FR 2077143
- FR 2393573
- DE 4424530
- DE 4424533
- DE 4402929
- DE 4420736
- WO 9523807 A
- WO 9407844 A
- EP 0646572 A
- WO 9516665 A
- FR 2673179
- EP 0227994 A
- WO 9424097 A
- WO 9410131 A
- US 4874554 A
- US 4137180 A
- EP 486135 A
- WO 9311103 A


### Littérature non-brevet citée dans la description


- Handbook of Surfactants. Blackie & Son, 1991, 116-178
- **P.D. DORGAN.** *Drug and Cosmetic Industry,* Décembre 1983, 30-33
- **WALTER NOLL.** Chemistry and Technology of Silicones. Academie Press, 1968